(19) **Europäisches Patentamt**
**European Patent Office**
**Office européen des brevets**

(11) **EP 1 124 599 B1**

(12) **EUROPEAN PATENT SPECIFICATION**

(45) Date of publication and mention
of the grant of the patent:
**24.07.2013 Bulletin 2013/30**

(51) Int Cl.:
*A61M 1/36* *(2006.01)*    *A61B 5/026* *(2006.01)*
*G01F 9/00* *(2006.01)*

(21) Application number: **99970926.4**

(22) Date of filing: **22.10.1999**

(86) International application number:
**PCT/SE1999/001915**

(87) International publication number:
**WO 2000/024440 (04.05.2000 Gazette 2000/18)**

(54) **DEVICE FOR MEASURING ACCESS FLOW BY DIALYSIS**

VORRICHTUNG ZUM MESSEN VON ZUGANGSFLUSS BEI DIALYSE

DISPOSITIF DE MESURE DU FLUX D'ACCES LORS DE DIALYSE

(84) Designated Contracting States:
**AT BE CH CY DE DK ES FI FR GB GR IE IT LI LU MC NL PT SE**

(30) Priority: **23.10.1998 US 105396 P**

(43) Date of publication of application:
**22.08.2001 Bulletin 2001/34**

(60) Divisional application:
**09165573.8 / 2 198 900**

(73) Proprietor: **Gambro Lundia AB**
**220 10 Lund (SE)**

(72) Inventors:
 • **STERNBY, Jan**
  **S-222 52 Lund (SE)**
 • **MISHKIN, Gary**
  **Potomac, MD 20854 (US)**
 • **ASBRINK, Perry**
  **S-215 82 Malmö (SE)**
 • **NILSSON, Eddie**
  **S-280 10 Sösdala (SE)**

(74) Representative: **Roberts, Mark Peter et al**
**J A Kemp**
**14 South Square**
**Gray's Inn**
**London WC1R 5JJ (GB)**

(56) References cited:
**EP-A1- 0 943 369    EP-A2- 0 773 035**
**US-A- 4 923 598    US-A- 4 995 268**
**US-A- 5 830 365**

 • **STERNBY J.: 'Urea sensors - A world of possibilities' ADVANCES IN RENAL REPLACEMENT THERAPY vol. 6, no. 3, July 1999, pages 265 - 272, XP002927491**
 • **LINDSAY R.M. ET AL.: 'Monitoring vascular access flow' ADVANCES IN RENAL REPLACEMENT THERAPY vol. 6, no. 3, July 1999, pages 273 - 277, XP002927492**
 • **YARAR D. ET AL.: 'Ultrafiltration method for measuring vascular access flow rates during hemodialysis' KIDNEY INTERNATIONAL vol. 56, no. 3, 1999, pages 1129 - 1135, XP002927493**
 • **DEPNER T.A. ET AL.: 'Access flow measured from recirculation of urea during hemodialysis with reversed blood lines' J. AM. SOC. NEPHROL. vol. 6, 1995, page 486, XP002927494**
 • **LINDSAY R.M. ET AL.: 'The estimation of hemodialysis access blood flow rates by a urea method is a poor predictor of access outcome' ASAIO JOURNAL vol. 44, 1998, pages 818 - 822, XP002927495**

**Description**

The present invention relates to a device which can be used in a method for measuring blood flow rate in a blood access. Blood is taken out from the body of a mammal to an extracorporeal blood circuit through a blood access, via needles or a catheter.

There are several types of treatments in which blood is taken out in an extracorporeal blood circuit. Such treatments involve, for example, hemodialysis, hemofiltration, hemodiafiltration, plasmapheresis, blood component separation, blood oxygenation, etc. Normally, blood is removed from a blood vessel at an access site and returned to the same blood vessel or at another location in the body.

In hemodialysis and similar treatments, an access site is commonly surgically created in the nature of a fistula. Blood needles are inserted in the area of the fistula. Blood is taken out from the fistula via an arterial needle and blood is returned to the fistula via a venous needle.

A common method of generating a permanent access site having capability of providing a high blood flow and being operative during several years and even tens of years, is the provision of an arterio-venous fistula. It is produced by operatively connecting the radial artery to the cephalic vein at the level of the forearm. The venous limb of the fistula thickens during the course of several months, permitting repeated insertion of dialysis needles.

An alternative to the arterio-venous fistula is the arterio-venous graft, in which a connection is generated from, for example, the radial artery at the wrist to the basilic vein. The connection is made with a tube graft made from autogenous saphenous vein or from polytetrafluorethylene (PTFE, Teflon). The needles are inserted in the graft.

A third method for blood access is to use a silicon, dual-lumen catheter surgically implanted into one of the large veins.

Further methods find use in specific situations, like a no-needle arterio-venous graft consisting of a T-tube linked to a standard PTFE graft. The T-tube is implanted in the skin. Vascular access is obtained either by unscrewing a plastic plug or by puncturing a septum of said T-tube with a needle. Other methods are also known.

During hemodialysis, it is desirable to obtain a constant blood flow rate of 150 - 500 ml/min or even higher, and the access site must be prepared for delivering such flow rates. The blood flow in an AV fistula is often 800 ml/min or larger, permitting delivery of a blood flow rate in the desired range.

In the absence of a sufficient forward blood flow, the extracorporeal circuit blood pump will take up some of the already treated blood entering the fistula via the venous needle, so called access or fistula recirculation, leading to poor treatment results.

The most common cause of poor flow with AV fistulas is partial obstruction of the venous limb due to fibrosis secondary to multiple venipunctures. Moreover, stenosis causes a reduction of access flow.

When there is a problem with access flow, it has been found that access flow rate often exhibit a long plateau time period with reduced but sufficient access flow, followed by a short period of a few weeks with markedly reduced access flow leading to recirculation and ultimately access failure. By constantly monitoring the evolution of the access flow during consecutive treatment sessions, it is possible to detect imminent access flow problems.

Several methods have been suggested for monitoring recirculation and access flow. Many of these methods involve injection of a marker substance in blood, and the resultant recirculation is detected. The methods normally involve measurement of a property in the extracorporeal blood circuit. Examples of such methods can be found in US 5,685,989, US 5,595,182, US 5,453,576, US 5,510,716, US 5,510,717, US 5, 312, 550, etc.

Such methods have the disadvantage that they cannot detect when the access flow has decreased to such an extent that recirculation is at risk, but only when recirculation prevails. Moreover, it is a drawback that injection of a substance is necessary.

A noninvazive technique that allows imaging of flow through AV grafts is color Doppler ultrasound. However, this technique requires expensive equipment.

EP 0 773 035 describes a method for operating a blood treatment device for determining haemodynamic parameters, like a fistula flow, during extracorporeal blood treatment.

The measurement of access flow rate necessitates the reversal of the flows in the extracorporeal circuit. A valve for such reversal is shown in i.a. US 5605630 and US 5894011. However, these valve constructions comprises dead ends in which blood may stand still for a long time and coagulate, which is a drawback.

An object of the present invention is to provide a device for measuring the access flow rate without interfering with the blood and without injecting a substance in blood.

Another object of the invention is to provide a device for measuring access flow rate without measuring on the blood in the extracorporeal blood circuit or in the access or blood vessel.

According to the invention, it is required to reverse the blood flow through the access. Thus, a further object of an embodiment of the invention is, provide a valve for reversing the blood flow.

A possible use of the invention is to provide a method for determining when the blood flow rate is so small that risk for recirculation prevails.

These objects are achieved with an apparatus as defined in the claims

Preferably, the calculation of the fluid flow rate in said flow access takes place by calculating the ratio between the first and the second variable and using the formula: Cd norm / Cd rev = 1 + K/Qa, in which Cd norm and Cd rev are values proportional to the concentrations of said substance in the dialysis fluid in the normal and reversed positions, respectively, and K is the clearance of the dialyzer and Qa is the access flow rate.

The blood flow access may be in a mammal for obtaining access to a blood vessel, such as a hemodialysis access in the nature of an arterio-venous shunt or fistula. In the latter case, the dialyzer clearance K is replaced by the effective dialyzer clearance Keff obtained by taking into account a cardiopulmonary recirculation and in the normal position.

The substance is preferably selected from the group of: urea, creatinine, vitamin B12, beta-two-microglobuline and glucose, or may be an ion selected from the group of: $Na^+$, $Cl^-$, $K^+$, $Mg^{++}$, $Ca^{++}$, $HCO_3^-$, acetate ion, or any combination thereof as measured by conductivity; and wherein said concentration is measured as the concentration difference between the outlet and the inlet of the dialyzer, if applicable.

It is possible to measure the actual concentration of the substance. However, since only the ratio between the concentrations in the normal and the reversed position, respectively, is needed, it is possible to measure a value which is proportional to the concentration of said substance, whereby said value is used in place of said concentration. Said property may be the blood concentration of said substance in the external circuit, either before or after the dialyzer. Alternatively, the relative whole body efficiency ($K_{wh}/V$) may be used, as explained in more detail below.

The effective clearance Keff may be obtained by the equation Keff = Qd * Cd / Cs, where Qd is the flow of dialysis fluid emitted from the dialyzer, Cd is the concentration of said substance in said dialysis fluid and Cs is the concentration of said substance in systemic venous blood.

A method of measuring the concentration (Cs) of said substance in systemic venous blood comprises the steps of: stopping the blood flow in the external flow circuit for a time period sufficient to allow the cardiopulmonary circulation to equalize; starting the blood flow in the external flow circuit with a slow speed to fill the arterial line with fresh blood before the measurement; and measuring the equalized concentration of said substance in the dialysis fluid at a low dialysate flow rate or at isolated ultrafiltration. It is advantageous to make the measurement of the effective clearance at the initiation of the treatment.

The concentration (Cs) of said substance in systemic venous blood may be estimated by: calculating a whole body mass of urea (Murea) in the body of the patient, estimating or measuring the distribution volume (V) of urea in the body of the patient; and estimating the concentration (Cs) of said substance in the blood by dividing the whole body mass of urea with the distribution volume. In this way, the mean concentration of urea in the whole body is obtained. However, the mean concentration in the whole body is slightly higher than the urea concentration in the systemic blood, except at the start of the treatment. Thus, this calculation should preferably be done or be extrapolated to the start of the treatment.

It is possible to discriminate between the condition when access or fistula recirculation has developed and not. A method for that purpose would be: changing the blood flow rate (Qb); monitoring the concentration of said substance in the dialysate emitted from the dialyzer; and detecting a possible fistula recirculation in the normal position by correlating a change in said concentration to said change of the blood flow rate.

Preferably, the blood flow rate is decreased and a corresponding decrease in the urea concentration is monitored, and the abscence of such a decrease being indicative of fistula recirculation.

Further objects, advantages and features of the invention appears from the following detailed description of the invention with reference to specific embodiments of the invention shown on the drawings, in which

Fig. 1 is a partially schematic view of a forearm of a patient provided with an AV fistula.

Fig. 2 is a schematic diagram of an extracorporeal dialysis circuit.

Fig. 3 is a schematic diagram of the blood flow circuit in a patient and in the attached extracorporeal blood circuit.

Fig. 4 is a schematic diagram similar to Fig. 3, but with the extracorporeal circuit in an alternative reversed position.

Fig. 5 is a schematic diagram of a blood flow circuit including a switch valve.

Fig. 6 is a diagram of the dialysis fluid urea concentration versus time, including a portion with reversed flow access according to the invention.

Fig. 7 is a schematic diagram similar to the diagram of Fig. 5 comprising an alternative valve arrangement.

Fig. 8 is schematic diagram similar to the diagram of Fig. 7 showing the valve arrangement in an idle postion.

Fig. 9 is schematic diagram similar to the diagram of Fig. 7 showing the valve arrangement in a reversed postion.

Fig. 10 is a schematic diagram similar to Fig. 5 with the pump in an alternative position.

Fig. 11 is a diagram showing calculations with relative whole body efficiency.

Fig. 12 is a cross-sectional view of a valve housing to be used in the schematic diagram of Figs. 5 and 7 to 10.

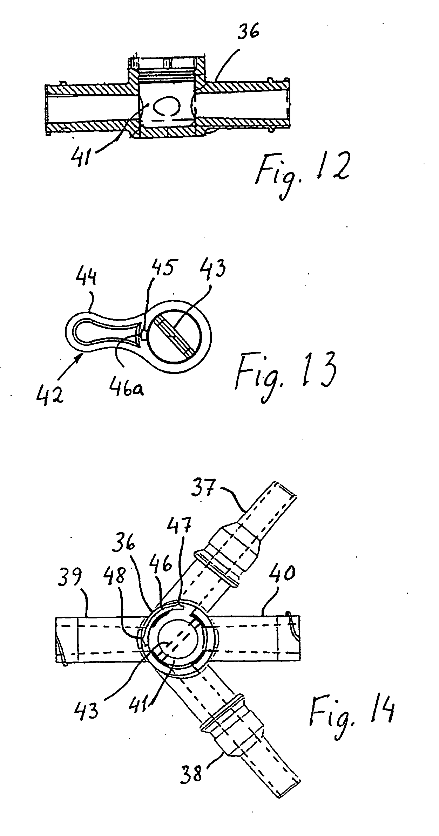

Figl 13 is a bottom view of a valve member intended to be inserted in the valve housing of Fig. 12.

Fig. 14 is a partially schematic plan view of the valve housing of Fig. 12.

For the purpose of this description, an access site is a site in which a fluid in a tube can be accessed and removed from and/or returned to the tube. The tube may be a blood vessel of a mammal, or any other tube in which a fluid is flowing. The access flow rate is the flow rate of the fluid in the tube or blood vessel immediately upstream of the access site or removal position. ,

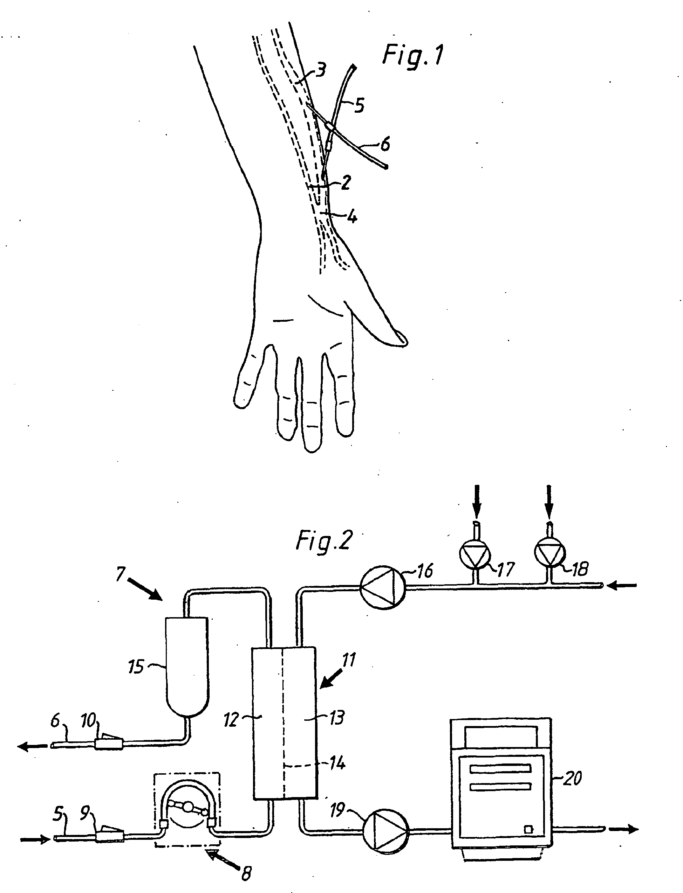

Fig. 1 discloses a forearm 1 of a human patient. The forearm 1 comprises an artery 2, in this case the radial artery, and a vein 3, in this case the cephalic vein. Openings are surgically created in the artery 2 and the vein 3 and the openings are connected to form a fistula 4, in which the arterial blood flow is cross-circuited to the vein. Due to the fistula, the blood flow through the artery and vein is increased and the vein forms an thickened area downstream of the connecting openings. When the fistula has matured after a few months, the vein is thicker and may be punctured repeatedly. Normally, the thickened vein area is called a fistula.

An arterial needle 5 is placed in the fistula, in the enlarged vein close to the connected openings and a venous needle 6 is placed downstream of the arterial needle, normally at least five centimeters downstream thereof.

The needles 5 and 6 are connected to a tube system 7, shown in Fig. 2, forming an extracorporeal circuit comprising a blood pump 8, such as a dialysis circuit. The blood pump propels blood from the blood vessel, through the arterial needle, the extracorporeal circuit, the venous needle and back into the blood vessel.

The extracorporeal blood circuit 7 shown in Fig. 2 further comprises an arterial clamp 9 and a venous clamp 10 for isolating the patient from the extracorporeal circuit should an error occur.

Downstream of pump 8 is a dialyzer 11, comprising a blood compartment 12 and a dialysis fluid compartment 13 separated by a semipermeable membrane 14. Further downstream of the dialyzer is a drip chamber 15, separating air from the blood therein.

Blood passes from the arterial needle past the arterial clamp 9 to the blood pump 8. The blood pump drives the blood through the dialyzer 11 and further via the drip chamber 15 and past the venous clamp 10 back to the patient via the venous needle. The drip chamber may comprise an air detector, adapted to trigger an alarm should the blood emitted from the drip chamber comprise air or air bubbles. The blood circuit may comprise further components, such as pressure sensors etc.

The dialysis fluid compartment 13 of the dialyzer 11 is provided with dialysis fluid via a first pump 16, which obtains dialysis fluid from a source of pure water, normally RO-water, and one or several concentrates of ions, metering pumps 17 and 18 being shown for metering such concentrates. The preparation of dialysis fluid is conventional and is not further described here.

An exchange of substances between the blood and the dialysis fluid takes place in the dialyzer through the semipermeable membrane. Notably, urea is passed from the blood, through the semipermeable membrane and to the dialysis fluid present at the other side of the membrane. The exchange may take place by diffusion under the influence of a concentration gradient, so called hemodialysis, and/or by convection due to a flow of liquid from the blood to the dialysis fluid, so called ultrafiltration, which is an important feature of hemodiafiltration or hemofiltration.

From the dialyses fluid compartment 13 of the dialyzer is emitted a fluid called the dialysate, which is driven by a second pump 19 via a urea monitor 20 to drain. The urea monitor continuously measures the urea concentration in the dialysate emitted from the dialyzer, to provide a dialysate urea concentration curve during a dialysis treatment. Such urea concentration curve may be used for several purposes, such as obtaining a total body urea mass, as described in WO 9855166, and to obtain a prediction of the whole body dialysis dose Kt/V as also described in said application.

As described above, the present invention can be used in a method of non-invasively measuring the access flow in the fistula immediately before the arterial needle, using the urea monitor and the dialysis circuit as shown in Fig. 2.

By measuring the dialysis urea concentration during normal dialysis and then reversing the positions of the needles and measuring the dialysis urea concentration with the needles in the reversed position, it is possible to calculate the blood flow in the blood access, without the addition of any substance to blood or the dialysis fluid.

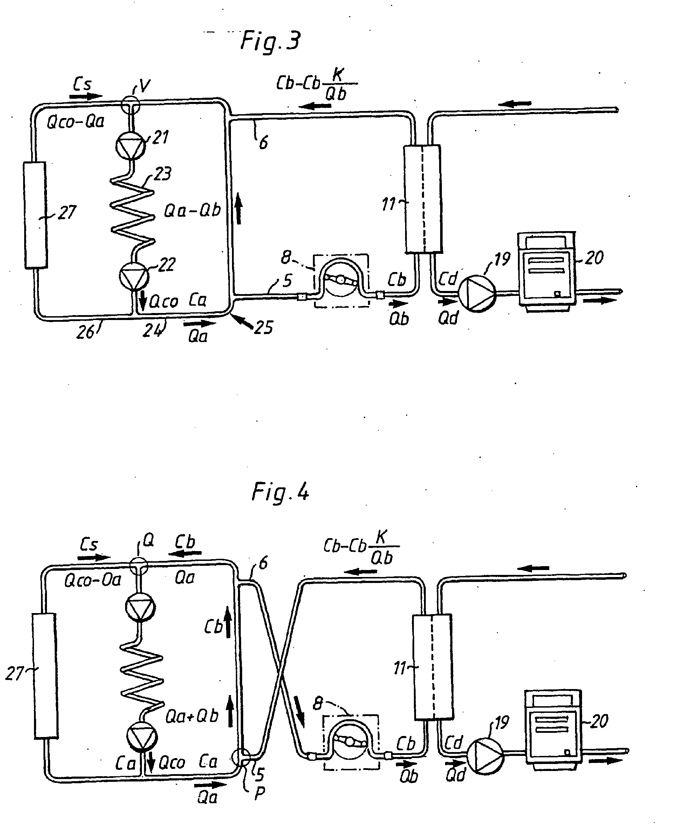

Fig. 3 shows a simplified schematic diagram of the blood vessel circuit of a patient and a portion of the dialysis circuit according to Fig. 2. The patient blood circuit comprises the heart, where the right chamber of the heart is symbolized by an upper pump 21 and the left chamber of the heart is symbolized by a lower pump 22. The lungs 23 are located between the upper and lower pump. From the outlet of the left chamber pump 22 of the heart, the blood flow divides into a first branch 24 leading to the access 25, normally in the left forearm of the patient, and a second branch 26 leading to the rest of the body, such as organs, other limbs, head, etc. symbolized by a block 27. Blood returning from the body from the organs etc., i.e. from block 27, combines with blood returning from the access and enters the right chamber pump 21.

The cardiac output flow rate is defined as Qco and the flow rate of the access is defined as Qa, which means that Qco - Qa enters the block 27. The venous blood returning from block 27 before being mixed with blood from the access, the systemic venous blood, has a urea concentration of Cs. The blood leaving the left chamber pump 22 has a urea concentration of Ca equal to that passing out to the access 25 as well as to the block 27.

For measuring the access flow rate, it is necessary to reverse the flow through the arterial and venous needles. One way of achieving that is to reverse the needles manually.

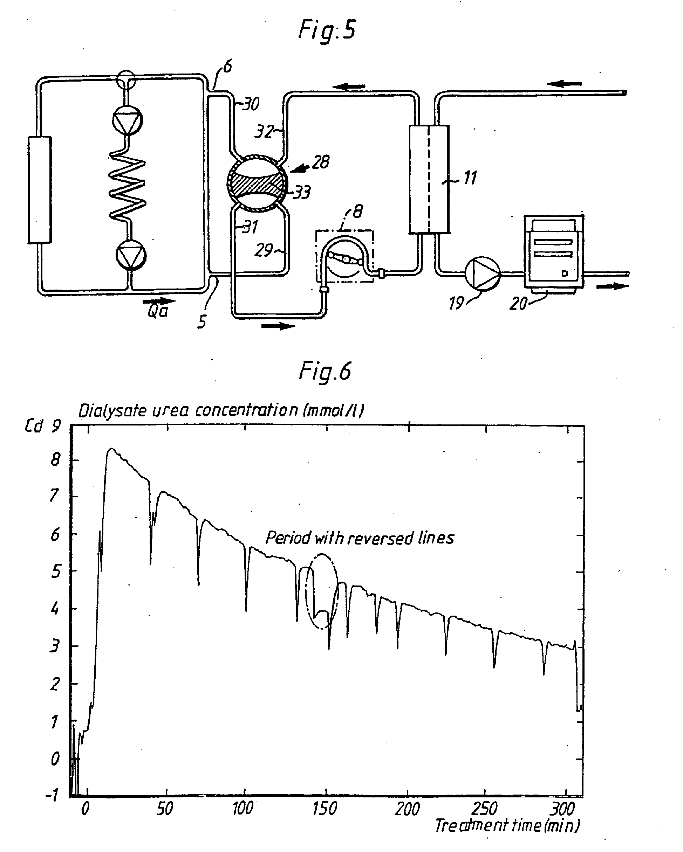

Alternatively, Fig. 5 shows a valve 28 for performing the same operation. The arterial needle 5 is connected to an arterial inlet line 29 of the valve and the venous needle 6 is connected to a venous inlet line 30 of the valve. The blood pump is connected to a first outlet line 31 of the valve and the returning blood from the dialyzer 11 is connected to a second outlet line 32 of the valve.

The valve comprises a valve housing and a pivotable valve member 33, which is pivotable from the normal position shown on the drawing to a reverse position pivoted 90° in relation to the normal position.

In the normal position shown in Fig. 5, the arterial needle 5 is connected to the blood pump 8 and the venous needle 6 is connected to the outlet of the dialyzer, via the drip chamber, see Fig. 2. In the reversed position, the arterial needle 5 is connected to the outlet of the dialyzer and the venous needle 6 is connected to the blood pump 8, as required.

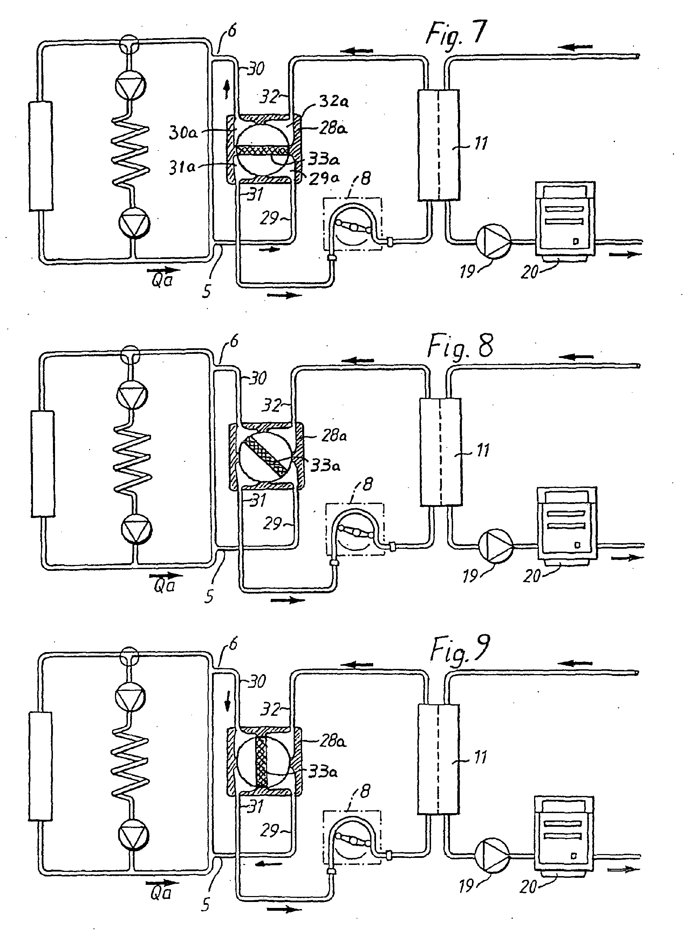

An alternative design of the valve arrangement is shown in Figs. 7, 8 and 9. In the embodiment of Fig. 7, the arterial line 29 is connected to an enlarged opening 29a and the venous outlet line 30 is connected to an enlarged opening 30a, the openings being arranged in the valve housing 28a diametrically opposite to each other. Two enlarged openings 31a and 32a are arranged in the valve housing 28a diametrically opposite each other and displaced 90° in relation to enlarged openings 29a and 30a. The pivotable valve member 33a is normally arranged as shown in Fig. 7 and forms a partition dividing the valve chamber in two semi-circular portions. The valve member has a width, which is smaller than the peripheral dimension of the enlarged openings. The valve member is pivotable 90° to a reverse position, shown in Fig. 9, in which the blood flows through the arterial and venous needles are reversed.

During its movement from the normal to the reversed position, the valve member 33a passes through an idle position shown in Fig. 8, in which all four enlarged openings are interconnected, because the width of the valve member is smaller than the peripheral dimension of the enlarged openings. By this idle position, harm to blood cells may be avoided. Such harm may be caused by high shear stresses which may occur if the inlet line 31 to the blood pump or the outlet line 32 from the dialyzer are completely occluded. By means of the idle position, another advantage is obtained, that the blood needles are not exposed to rapid change of flows, which in some instances even may result in dislocation of the needles. When the valve member is moved from the normal position to the idle position, the flow through the needles change from the normal flow of, for example, 250 ml/min to essentially zero flow. The valve member may be placed in the idle position for some seconds. Then, the valve member is moved to the reversed position, and the flows through the needles is changed from essentially zero flow to -250 ml/min. In this way, a more gentle switch between normal and reversed flows may be obtained.

It is noted, that the positions of the openings and the valve member may be different so that the pivotal movement may be less than or more than 90°. Moreover, the openings need not be arranged diametrically in order to achieve the desired operation. Furthermore, the dimensions of the enlarged openings in relation to the tubes and lines are not in scale, but the diameter of the enlarged openings is rather of the same dimension as the tube inner diameter, as appears more clearly below.

It is noted that the valve is constructed to have as few dead end portions as possible, in which the blood may stand still and coagulate. From the drawing, it is appreciated that no portion of the valve has a dead end construction in any position of the valve body.

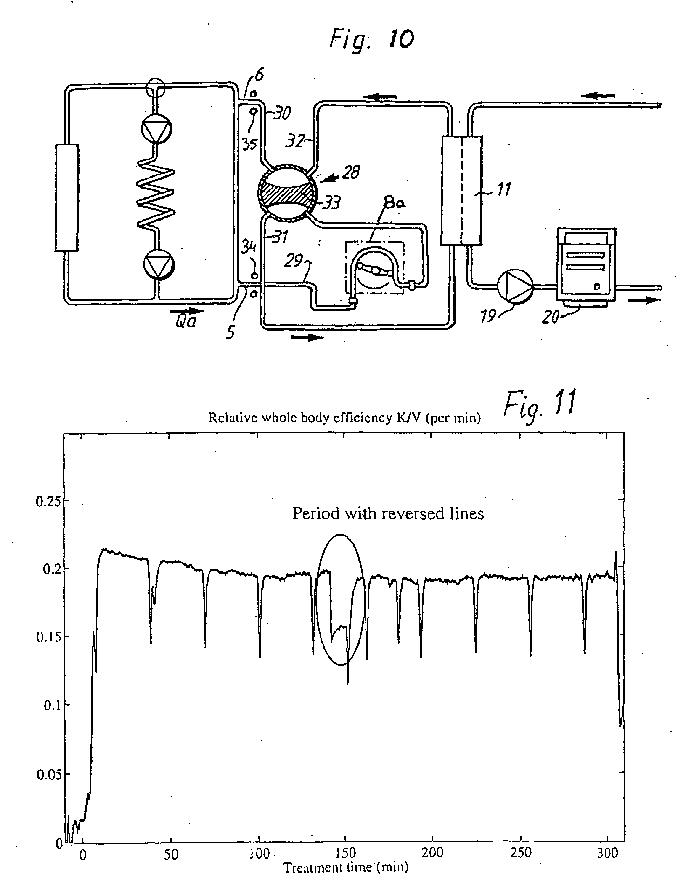

Furthermore, another schematic diagram incorporating a valve is shown in Fig. 10. Fig. 10 differs from Fig. 5 only in the placement of the pump 8a, which in the embodiment according to Fig. 10 is placed between the arterial needle 5 and the valve 28. In this manner, the pressure across the valve body 33 is less compared to the embodiment according to Fig. 5. The operation is somewhat different. The blood pump is stopped, and the valve is put in the reversed position. Finally, the pump is started and pumping the blood in the opposite direction by reversing the rotational direction of the pump.

In order to ascertain that no air is introduced into the patient in either position of the valve, it may be advantageous to add an air detector 34 and 35 immediately before each of the arterial and venous needle, or at least before the arterial needle. The air detectors trigger an alarm should they measure air bubbles in the blood given back to the blood vessel. Normally, the air detector in the drip chamber is sufficient for this purpose.

The detailed construction of a valve intended to be used in the present invention, is disclosed in Figs. 12, 13 and 14. The valve comprises a valve housing 36 comprising two inlet connectors and two outlet connectors. All four connectors open into cylindrical valve chamber 41, the four openings being displaced 90° in relation to each other.

As shown in Fig. 14, the valve comprises a blood inlet connector 37 connected to the arterial needle 5 and a blood outlet connector 38 connected to the venous needle 6. The connector portions are arranged as male Luer connectors to be connected to flexible tubes ending with a female Luer connector.

Furthermore, the valve comprises a circuit outlet connector 39 connected to the blood pump 8 and a circuit inlet connector 40 connected to the dialyzer outlet. The connector portions 39 and 40 are arranged as female Luer connectors to mate with male Luer connectors of the circuit.

As appears from Fig. 12, the cylindrical valve chamber 41 is closed at the bottom. From the top, a valve member 42 may be introduced into the cylindrical valve chamber. The valve member 42 comprises a valve partition 43 as appears from Fig. 13.

The valve member also comprises an operating wing 44, by means of which the valve member may be pivoted 90° between a normal position, in which the valve partition 43 is situated as shown by dotted lines in Fig. 14, and a reversed position. The pivotal movement is limited by a shoulder 45 of the valve member 42, which cooperates with a groove 46 in the valve housing. The shoulder 45 is provided with a protrusion 46a which cooperates with two recesses 47 and 48 in the normal position and reverse position, respectively, to maintain the valve member in either position. The groove 46 may be provided with a third recess (not shown in the drawing) in order to define said idle position. Such a third recess is positioned in the middle between the two recesses 47 and 48.

The valve member and housing are provided with suitable sealings to ensure safe operation. The operation of the valve is evident from the above description.

By studying the theoretical dialysate urea concentrations resulting from a given dialyzer clearance K, a given access blood flow Qa and a given blood urea concentration Cs in the systemic venous blood returning from the body, it is found that the effective urea clearance Keff of the dialyzer, taking the cardiopulmonary recirculation into account, is needed for the calculation of access flow. The effective clearance can be measured, for example as described in EP 658 352 .

Alternatively, the effective clearance can be calculated from simultaneous systemic venous blood Cs and dialysate Cd measurements of urea concentrations, such as by blood samples.

The systemic blood urea concentration Cs may be measured by the so called stop flow - slow flow technique, where the blood flow is substantially stopped for a couple of minutes to allow the cardiopulmonary recirculation to equalize. Thereafter, the pump is run slowly to fill the arterial line with fresh blood before taking the blood sample. The urea concentration in the so obtained blood sample is equal to the urea concentration Cs in the systemic venous blood returning from the body to the heart.

Alternatively to taking a blood sample, the dialysis fluid flow at the other side of the membrane is stopped and the slowly flowing blood is allowed to equalize with the dialysate at the other side of the membrane, whereupon the urea concentration of the dialysate is measured to obtain the systemic venous blood urea concentration Cs.

A further method to obtain effective clearance is described in WO 9929355. According to the invention described in WO 9929355, the systemic blood concentration Cs is measured before or at the initiation of the treatment, for example by stop flow - slow flow technique with blood sample or equilisation as described above. After obtaining valid dialysate urea concentration values Cd from a urea monitor connected to the dialysator outlet line, the initial dialysate urea concentration $C_{dinit}$ at the start of the treatment is extrapolated by the dialysate urea curve obtained.

A still further method of obtaining systemic blood urea concentration Cs is to calculate the urea mass $M_{wh}$ in the whole body and extrapolate the urea mass to the start of the treatment. By dividing the whole body urea mass $M_{wh}$ with the distribution volume V, the systemic blood urea concentration Cs at the start of the treatment is obtained.

By dividing the dialysate urea concentration Cd with the systemic blood urea concentration Cs and multiplicating with the dialysate flow rate Qd, the effective clearance Keff is obtained. It is advantageous to measure the effective clearance Keff at the initiation of the treatment.

Furthermore, in the method in the which the invention may be used, the blood flows in the arterial and venous needles are reversed. The dialysate urea concentrations in the two cases with normal position of the needles and with reverse position of the needles may be calculated as follows, with reference to Figs. 3 and 4.

The blood urea concentration Cs in the venous blood returning from the body is assumed unchanged when the lines are reversed, and the dialyzer clearance K is also assumed unchanged. For simplicity ultrafiltration is assumed to be zero, but it is also possible to handle a nonzero UF.

The following notations are used:

Qco — Cardiac Output
Qa — Access flow
Qb — Blood flow in extracorporeal circuit
Qd — Dialysate flow
K — Dialyzer clearance
Keff — Effective dialyzer clearance
Cs — Blood urea concentration in systemic venous blood returning from the body
Ca — Blood urea concentration in the access
Cb — Blood urea concentration at the dialyzer inlet
Cd — Dialysate urea concentration

The definition of clearance is:

$$K = (\text{removed urea}) / Cb = Qd * Cd / Cb \qquad (1)$$

Consider first the case in which Qa > Qb and the needles are in the normal position. In this case Cb = Ca.
Removal from blood must equal appearance in the dialysate so that

$$K * Ca = Qd * Cd \qquad (2)$$

A mass balance for urea at the point V, see Fig. 3, when mixing the venous return blood with the blood from the access gives:

$$Ca * Qco = Cs * (Qco - Qa) + Ca * (Qa - K) \qquad (3)$$

Thus, we obtain a relation between Ca and Cs.
By combining equations 2 and 3 we obtain:

$$Cd = (K/Qd) * Cs / [1 + K/(Qco - Qa)] \qquad (4)$$

The definition of effective clearance Keff implies that Cs should be used in the denominator instead of Cb as normally used in dialyzer clearance, which means that

$$Keff = K * (Cb / Cs) = K / [1 + K/(Qco - Qa)] \qquad (5)$$

If we now turn to the case with reversed lines, see Fig. 4, we still have that what is removed from the blood must enter the dialysate, so that in this case

$$K * Cb = Qd * Cd \qquad (6)$$

The flow in the fistula between the needles will be Qa + Qb and we can calculate the blood urea concentration at the dialyzer inlet from a urea mass balance at the point P where the dialyzed blood enters the access again

$$Cb * (Qb - K) + Ca * Qa = Cb * (Qb + Qa) \qquad (7)$$

We also have the mass balance at the point Q where the venous return blood meets the dialyzed blood in the access return flow:

$$Ca * Qco = Cs * (Qco - Qa) + Cb * Qa \qquad (8)$$

By eliminating Ca and Cb we get

$$Cd = (K/Qd) * Cs / [1 + (Qco/Qa) * K / (Qco - Qa)] \quad (9)$$

Since Cs, K and Qd in the two cases are unchanged, it is possible to obtain the ratio of dialysate urea concentrations:

$$Cd\ norm / Cd\ rev = 1 + (K/Qa) / [1 + K/(Qco-Qa)] =$$
$$= 1 + Keff/Qa \quad (10)$$

In practice, the two dialysate urea concentrations are probably best found by a curve fit to the dialysate urea curves before and after the switch of lines, with an extrapolation to the time of switching from the respective side, see Fig 6, which shows the urea concentration Cd of the dialysate during a normal hemodialysis treatment.

During a time period of about 10 minutes, marked with a ring in Fig. 6, the arterial and venous needles are reversed. After a initial time period for allowing the urea monitor to measure accurately, the urea concentration with reversed lines is appr. 0.8 times the original urea concentration, which means that Cdnorm / Cdrev = 1.25. Thus, if Keff is 200 ml/min, as measured with the needles in the normal position or estimated as described above, the access flow is 800 ml/min.

The effective clearance may also be obtained as a rough estimate from blood and dialyzer flows and dialyzer characteristics, e.g. from the dialyzer data sheet.

In the present specification, there is used three different clearances, namely dialyzer clearance, effective clearance and whole body clearance. If dialyzer clearance is 250 ml/min for a certain blood flow rate and dialysate flow rate, the effective clearance is normally 5 to 10% lower, such as 230 ml/min. The whole body clearance is still 5 to 15% lower, such as 200 ml/min. The dialyzer clearance is the clearance as measured directly on the dialyzer. The effective clearance is the clearance also taking into account the cardio-pumonary recirculation. Finally, the whole body clearance is the effective clearance further taking into account other membranes in the body restricting the flow of urea from any part of the body to the dialysate. The concept of whole body clearance is described in WO 9855166.

The effective clearance used in the formula may also be obtained from a measurement according to the method described in EP 658 352 mentioned above, with the needles in the normal position. This will give a measure of the effective plasma water urea clearance, which then has to be converted to whole blood clearance. The method of EP 658 352 essentially comprises that the conductivity of the dialysis fluid upstream of the dialyzer is increased by for example 10% and then returned to the original value. The result at the outlet side of the dialyzer is measured and reults in a measure of the effective clearance Keff of the dialyzer.

Alternatively, the effective clearance may be calculated according to equation Keff = Qd * Cd / Cs. The systemic venous urea concentration may be measured at the same time as the dialysate urea concentration Cd, or by the methods described above.

Another method would be to use the value of total body urea mass Murea obtained by the method according to WO 9855166, mentioned above. By obtaining the urea distribution volume V by Watson's formula or any other method, the venous urea concentration would be approximately:

$$Cs = Murea / V \quad (11)$$

In the method of WO 9855166, the relative whole body efficiency of the dialyzing process $K_{wb}/V$ is obtained. Note, that whole body clearance is used, as indicated by the subscript wb. According to said WO 9855166, urea concentration is proportional to the relative whole body efficiency according to the formula:

$$K_{wb}/V = (Q_d \cdot c_d) / m \quad (12)$$

Thus, if $(K_{Wb}/V)$ is used instead of Cd in the above equation (10), a similar result is obtained, if it is presumed that m is constant, i.e. the measurement must be extrapolated to the same time instance:

$$(K_{wb}/V) \text{ norm } / (K_{wb}/V) \text{ rev } = 1 + Keff/Qa \qquad (13)$$

As is mentioned in said WO 9855166, it is possible to calculate the relative whole body efficiency only from dialysate urea measurement. Since we are interested only in the ratio in the normal and reversed position, we do not need to calculate the actual $K_{wh}$.

Fig. 11 shows a plot of the relative whole body efficiency K/V ($min^{-1}$). The period with reversed lines is shown inside a circle. In all other respects, the same discussion applies as is given above.

The calculations above assume that the extracorporeal blood flow rate Qb does not exceed the access flow rate Qa. If this is the case there will be access recirculation and the flow in the access will be reversed when the needles are in the normal position. The calculation of dialysate urea concentration is unchanged for the needles in reversed position, but has to be modified for the needles in normal position. Calculations corresponding to those above show that the ratio above between dialysate urea concentrations for normal and reversed needle positions will be:

$$Cd \text{ norm } / Cd \text{ rev } = 1 + Keff / Qb \qquad (14)$$

where Keff is the effective clearance with the effect of recirculation included, that is with the needles in the normal position.

The only difference is that the calculation will now give the extracorporeal blood flow Qb instead of the access flow. This blood flow is known, so in practice this means that when the result is an access flow rate Qa close to the blood flow rate Qb, recirculation should be suspected, and this always means that the access has to be improved.

Keff/Qb is a figure lower than one, normally for example 0.6 - 0.9. Keff/ Qa should be considerably lower, for example 0.1 - 0.4. Thus, when Cd norm/Cd rev approaches or is lower than a predetermined number, such as 1.2 or 1.5, further calculations should be done for determining if access recirculation is present.

A simple procedure is to decrease the blood flow Qb somewhat. If the dialysate concentration then decreases, this means that there is no access or fistula recirculation at least at the lower blood flow.

The above calculations can also be made for the situation where ultrafiltration is present. However, it is a simple measure to reduce the ultrafiltration to zero during the measurement interval. Moreover, the error induced by ultrafiltration is small and may be neglected.

The measurement should be performed during a time interval, which is considerably larger than 30 seconds so that cardio-pulmonary recirculation has been developed. The measurement time for obtaining valid results may be 5 minutes with the needles reversed, while measurements with the needles in correct position may be done in 5 minutes or contiguously during the treatment.

The method is also applicable to the methods of treatment comprising infusion of a dialysis solution to the blood before or after the dialyzer, called hemofiltration and hemodiafiltration. The result is the same as given above.

If the access is a venous catheter, there is no cardio-pulmonary recirculation and the calculations becomes simpler. The result is the same, except that the effective clearance Keff is replaced by the dialyzer clearance K, since the systemic venous urea concentration Cs becomes the same as the dialyzer inlet urea concentration Cb.

It should be noted that all flow rates, clearances and urea concentrations in the calculations relate to whole blood. Approximately 93% of plasma is water, depending on the protein concentration, and about 72% of erythrocytes is water. Depending on the hematocrit value, the blood water volume is 10 - 13 % lower than the volume of whole blood, see for example Handbook of Dialysis, Second Edition, John T. Daugirdas and Todd. S Ing, 1994, page 18.

The effective urea clearance obtained according to EP 658 352 relates to blood water, and must therefore be increased by 10 - 13 % before being used in the present formulas. Blood urea concentration values obtained from a laboratory relate in general to plasma, and must therefore be decreased by about 7% in order to relate to whole blood.

Alternatively, all urea concentrations, flow rates and clearances may be used as relating to blood water. The effective clearance is then used unchanged, but the calculated access flow will relate to blood water, and has to be increased by 10 - 13 % to relate to whole blood.

The invention has been described above with reference to use in the human body and using urea as a marker for measuring access flow. However, any other substance present in blood and which can be measured at the dialysate side of the dialyzer may be used according to the invention, such as creatinine, vitamin B12, beta-two-microglobuline, NaCl or any combination of ions. Another alternative is to measure conductivity.

It is also possible to measure a property proportional to the concentration, since it is the ratio that is involved in the equations. Thus, urea concentration may be measured by measuring conductivity differences after passing the urea containing fluid through a urease column, and such conductivity difference can be used directly in place of the concentration values in the equations.

Other indirect methods of measuring any of the above-mentioned substances concentrations may be used as long as the measurements are made at the dialysate side of the dialyzer. Another alternative is to measure the blood urea concentrations by any known method, either before or after the dialyzer, since these concentrations are proportional to the concentrations in the formulas.

The invention has been described above with reference to use in the human body. However, the invention can be used in any tube system where a fluid is passed and a portion thereof is taken out for dialysis, such as in beer or wine production.

**Claims**

1. An apparatus for extracorporeal blood treatment comprising:

    • a dialyzer (11) having a first and a second compartments (12, 13) separated by a semi-permeable membrane;
    • a blood circuit having:

        a blood removal line (29) for connecting, in a first operative condition, an inlet of the first compartment (12) to a vascular access of a patient at a first connecting point (5), and
        a blood return line (30) for connecting, in the first operative condition, an outlet of the first compartment (12) to the vascular access of the patient at a second connecting point (6), wherein the first and second connecting points are spaced apart in the direction of flow;

    • a dialysis liquid circuit having:

        a supply line for supplying a dialysis liquid to an inlet of the second compartment (13), and
        a waste line for draining a waste liquid from an outlet of the second compartment (13),

    the apparatus being **characterized in that** it comprises:

        • a reversal means (28) for switching the connection of the blood removal line (29) and blood return line (30) to the vascular access so that, in a second operative condition, the first connecting point (5) is in fluid communication with the outlet of the first compartment (12) and the second connecting point (6) is in fluid communication with the inlet of the first compartment (12);
        • a measuring means (20) for measuring, in the first operative condition, a first value (Cd norm) of a property in the waste liquid, and for measuring, in the second operative condition, a second value (Cd rev) of the property in the waste liquid, the property being essentially proportional to the concentration of a substance in the waste liquid; and
        • a calculating means for calculating a fluid flow rate (Qa) in the vascular access from the first and second measured values (Cd norm, Cd rev) of the property in the waste liquid.

2. The apparatus according to claim 1, wherein the substance is selected from the group comprising urea, creatinine, vitamin B12, beta-two-microglobuline and glucose.

3. The apparatus according to claim 1, wherein the substance is an ion selected from the group comprising $Na^+$, $Cl^-$, $K^+$, $Mg^{++}$, $Ca^{++}$, $HCO_3^-$, acetate ion, or any combination thereof.

4. The apparatus according to one of the claims 1 to 3, wherein the property is the conductivity of the dialysis liquid.

5. The apparatus according to one of the claims 1 to 3, wherein the property is the difference in the concentration of the substance between the outlet and the inlet of the dialyzer (11).

6. The apparatus according to one of the claims 1 to 3, wherein the property is either the concentration of the substance in the blood or the relative whole body efficiency (Kwb/V) defined as the ratio'of the whole body clearance Kwb to the urea distribution volume V.

7. The apparatus according to one of the claims 1 to 6, wherein calculating the flow rate (Qa) of the liquid in the vascular access takes into account a parameter (K, Keff) indicative of the transfer of the substance through the semi-permeable membrane (14).

8. The apparatus according to claim 7, wherein the parameter indicative of the transfer of the substance through the semi-permeable membrane (14) is the clearance (K) of the dialyzer (11) for the substance.

9. The apparatus according to claim 8, wherein the flow rate (Qa) of the liquid in the tube is calculated using the equation:

$$\text{Cd norm / Cd rev} = 1 + K/Qa$$

10. The apparatus according to claim 7, wherein the tube is a blood flow access for hemodialysis such as an arterio-venous shunt or a fistula, and wherein the parameter indicative of the transfer of the substance through the semi-permeable membrane (14) is the effective dialyzer clearance Keff, which takes taking into account a cardiopulmonary recirculation.

11. The apparatus according to claim 10, wherein the flow rate (Qa) of the liquid in the blood flow access is calculated using the equation:

$$\text{Cd norm / Cd rev} = 1 + Keff/Qa$$

12. The apparatus according to claim 11, wherein the effective dialyzer clearance Keff is calculated using the equation:

$$\text{Keff} = Qd * Cd / Cs$$

In which Qd is the flow rate of the dialysis liquid downstream of the dialyzer (11), Cd is the concentration of the substance in the dialysis liquid downstream of the dialyzer (11), and Cs is the concentration of the substance in systemic venous blood.

13. The apparatus according to claim 12, further comprising means for measuring the concentration (Cs) of the substance in systemic venous blood, including:

• means (8, 8a) for stopping the blood flow in the blood circuit for a time period sufficient to allow the cardiopulmonary circulation to equalize and stopping the flow of dialysis liquid;
• means (8, 8a) for starting the blood flow in the blood circuit with a slow speed to fill the arterial line with fresh blood before the measurement;
• means (20) for measuring the equalized concentration of the substance in the waste liquid at a slow flow rate or at isolated ultrafiltration.

14. The apparatus according to claim 12, further comprising means for estimating the concentration (Cs) of the substance in systemic venous blood, including:

• means for calculating a whole body mass of urea (Murea) in the body of the patient,
• means for estimating or measuring the distribution volume (V) of urea in the body of the patient;
• means for estimating the concentration (Cs) of the substance in the blood by dividing the whole body mass of urea with the distribution volume.

15. The apparatus according to one of the claims 1 to 14, further comprising:

• means (8, 8a) for changing the blood flow rate (Qb);
• means (20) for monitoring the concentration of said substance in the waste liqu.id;
• means for detecting a possible fistula recirculation in the first position by correlating a change in said concentration to the change of the blood flow rate.

16. The apparatus according to claim 15, comprising means (8, 8a) for decreasing the blood flow rate and means (20) for monitoring a corresponding decrease in the substance concentration, whereby the absence of such a decrease is indicative of fistula recirculation.

**17.** The apparatus according to one of the claims 1 to 16, wherein the reversal means comprises a valve means (28, 28a; 36, 41) connected to the blood removal line (29) and to the blood returning line (30) for selectively inversing the connection of the blood removal line (29) and of the blood return line (30) to the inlet and to the outlet of the first compartment (12) of the dialyzer (11), the valve means (28, 28a; 36, 41) comprising two inlet and two outlet openings (29a, 30a, 31a, 32a; 37, 38, 38, 40) and a valve member (33, 33a; 43) that is so arranged as to selectively adopt an idle position in which all four inlet and outlet openings (29a, 30a, 31a, 32a; 37, 38, 38, 40) are interconnected.

**18.** The apparatus according to claim 17, further comprising an air detector (34, 35) arranged between the valve means (28, 28a; 36, 41) and the end to be connected to the vascular access of a patient of at least one of the blood removal line (29) and the blood returning line (30).

**Patentansprüche**

**1.** Vorrichtung für die Blutbehandlung außerhalb des Körpers, umfassend:

- einen Dialysator (11) mit einer ersten und einer zweiten Kammer (12, 13), die durch eine halbdurchlässige Membran getrennt sind;
- einen Blutkreislauf, aufweisend:

eine Blutentnahmeleitung (29) zum Verbinden, in einem ersten Betriebszustand, eines Einlasses der ersten Kammer (12) mit einem Gefäßzugang eines Patienten an einem ersten Verbindungspunkt (5), und eine Blutrückflussleitung (30) zum Verbinden, in dem ersten Betriebszustand, eines Auslasses der ersten Kammer (12) mit dem Gefäßzugang des Patienten an einem zweiten Verbindungspunkt (6), wobei der erste und zweite Verbindungspunkt in der Fließrichtung beabstandet sind;

- einen Dialysierflüssigkeitskreislauf, aufweisend:

eine Zuführleitung zum Zuführen einer Dialysierflüssigkeit zu einem Einlass der zweiten Kammer (13), und eine Abfallstoffleitung zum Ableiten einer Abfallstoffflüssigkeit aus einem Auslass der zweiten Kammer (13),

wobei die Vorrichtung **dadurch gekennzeichnet ist, dass** sie Folgendes umfasst:

- ein Umkehrmittel (28) zum Umschalten der Verbindung der Blutentnahmeleitung (29) und Blutrückflussleitung (30) mit dem Gefäßzugang, sodass, in einem zweiten Betriebszustand, der erste Verbindungspunkt (5) über eine Flüssigkeit mit dem Auslass der ersten Kammer (12) in Verbindung steht und der zweite Verbindungspunkt (6) über eine Flüssigkeit mit dem Einlass der zweiten Kammer (12) in Verbindung steht;
- ein Messmittel (20) zum Messen, in dem ersten Betriebszustand, eines ersten Werts (Cd norm) einer Eigenschaft in der Abfallstoffflüssigkeit und zum Messen, in dem zweiten Betriebszustand, eines zweiten Werts (Cd rev) der Eigenschaft in der Abfallstoffflüssigkeit, wobei die Eigenschaft im Wesentlichen proportional zur Konzentration einer Substanz in der Abfallstoffflüssigkeit ist; und
- ein Berechnungsmittel zum Berechnen eines Flüssigkeitsdurchflusses (Qa) in dem Gefäßzugang aus dem ersten und zweiten Messwert (Cd norm, Cd rev) der Eigenschaft in der Abfallstoffflüssigkeit.

**2.** Vorrichtung nach Anspruch 1, wobei die Substanz aus der Gruppe ausgewählt ist, die Harnstoff, Kreatinin, Vitamin B12, Beta-2-Mikroglobulin und Glucose umfasst.

**3.** Vorrichtung nach Anspruch 1, wobei die Substanz ein Ion ist, das aus der Gruppe ausgewählt ist, die Na$^+$, Cl$^-$, K$^+$, Mg$^{++}$, Ca$^{++}$, HCO$_3^-$, das Acetat-Ion oder jede beliebige Kombination daraus umfasst.

**4.** Vorrichtung nach einem der Ansprüche 1 bis 3, wobei die Eigenschaft die Leitfähigkeit der Dialysierflüssigkeit ist.

**5.** Vorrichtung nach einem der Ansprüche 1 bis 3, wobei die Eigenschaft der Unterschied der Konzentration der Substanz zwischen dem Auslass und dem Einlass des Dialysators (11) ist.

**6.** Vorrichtung nach einem der Ansprüche 1 bis 3, wobei die Eigenschaft entweder die Konzentration der Substanz im Blut oder die relative Ganzkörpereffizienz (Kwb/V) ist, die als das Verhältnis der Ganzkörper-Clearance Kwb zum Harnstoff-Verteilungsvolumen V definiert ist.

7. Vorrichtung nach einem der Ansprüche 1 bis 6, wobei bei der Berechnung des Durchflusses (Qa) der Flüssigkeit im Gefäßzugang ein Parameter (K, Keff) berücksichtigt wird, der den Transport der Substanz durch die halbdurchlässige Membran (14) angibt.

8. Vorrichtung nach Anspruch 7, wobei der Parameter, der den Transport der Substanz durch die halbdurchlässige Membran (14) angibt, die Clearance (K) des Dialysators (11) für die Substanz ist.

9. Vorrichtung nach Anspruch 8, wobei der Durchfluss (Qa) der Flüssigkeit in der Röhre berechnet wird unter Verwendung der Gleichung:

$$Cd\ norm\ /\ Cd\ rev = 1 + K/Qa$$

10. Vorrichtung nach Anspruch 7, wobei die Röhre ein Zugang zum Blutstrom für die Hämodialyse wie ein arteriovenöser Shunt oder eine Fistel ist und wobei der Parameter, der den Transport der Substanz durch die halbdurchlässige Membran (14) angibt, die effektive Dialysator-Clearance Keff ist, bei der eine kardiopulmonale Rezirkulation berücksichtigt wird.

11. Vorrichtung nach Anspruch 10, wobei der Durchfluss (Qa) der Flüssigkeit in dem Zugang zum Blutstrom berechnet wird unter Verwendung der Gleichung:

$$Cd\ norm\ /\ Cd\ rev = 1 + Keff/Qa$$

12. Vorrichtung nach Anspruch 11, wobei die effektive Dialysator-Clearance Keff berechnet wird unter Verwendung der Gleichung:

$$Keff = Qd * Cd\ /\ Cs$$

in der Qd der Durchfluss der Dialysierflüssigkeit stromab des Dialysators (11) ist, Cd die Konzentration der Substanz in der Dialysierflüssigkeit stromab des Dialysators (11) ist und Cs die Konzentration der Substanz im systemischen venösen Blut ist.

13. Vorrichtung nach Anspruch 12, ferner umfassend Mittel zum Messen der Konzentration (Cs) der Substanz im systemischen venösen Blut, aufweisend:

- Mittel (8, 8a) zum Anhalten des Blutstroms in dem Blutkreislauf über einen ausreichenden Zeitraum, um den Ausgleich der kardiopulmonalen Zirkulation zuzulassen und Anhalten des Dialysierflüssigkeitsstroms;
- Mittel (8, 8a) zum erneuten Laufenlassen des Blutstroms in dem Blutkreislauf mit einer langsamen Geschwindigkeit, um die arterielle Leitung vor der Messung mit frischem Blut zu füllen;
- Mittel (20) zum Messen der ausgeglichenen Konzentration der Substanz in der Abfallstoffflüssigkeit bei einem langsamen Durchfluss oder bei isolierter Ultrafiltration.

14. Vorrichtung nach Anspruch 12, ferner umfassend Mittel zum Bestimmen der Konzentration (Cs) der Substanz im systemischen venösen Blut, aufweisend:

- Mittel zum Berechnen einer Ganzkörpermasse an Harnstoff (MHarnstoff) im Körper des Patienten,
- Mittel zum Bestimmen oder Messen des Verteilungsvolumens (V) von Harnstoff im Körper des Patienten;
- Mittel zum Bestimmen der Konzentration (Cs) der Substanz im Blut durch Teilen der Ganzkörpermasse an Harnstoff durch das Verteilungsvolumen.

15. Vorrichtung nach einem der Ansprüche 1 bis 14, ferner umfassend:

- Mittel (8, 8a) zum Ändern des Blutdurchflusses (Qb);
- Mittel (20) zum Überwachen der Konzentration der Substanz in der Abfallstoffflüssigkeit;
- Mittel zum Erfassen einer möglichen Fistelrezirkulation in der ersten Position durch Korrelieren einer Änderung der Konzentration mit der Änderung des Blutdurchflusses.

**16.** Vorrichtung nach Anspruch 15, umfassend Mittel (8, 8a) zum Verringern des Blutdurchflusses und Mittel (20) zum Überwachen eines entsprechenden Rückgangs der Substanzkonzentration, wobei das Fehlen eines derartigen Rückgangs eine Fistelrezirkulation anzeigt.

**17.** Vorrichtung nach einem der Ansprüche 1 bis 16, wobei das Umkehrmittel ein Ventilmittel (28, 28a; 36, 41) umfasst, das mit der Blutentnahmeleitung (29) und mit der Blutrückflussleitung (30) verbunden ist, um die Verbindung der Blutentnahmeleitung (29) und der Blutrückflussleitung (30) mit dem Einlass und mit dem Auslass der ersten Kammer (12) des Dialysators (11) gezielt umzukehren, wobei das Ventilmittel (28, 28a; 36, 41) zwei Einlass- und zwei Auslassöffnungen (29a, 30a, 31 a, 32a; 37, 38, 38, 40) und ein Ventilelement (33, 33a; 43) umfasst, das so angeordnet ist, dass es gezielt eine Ruhestellung einnimmt, in der alle vier Einlass- und Auslassöffnungen (29a, 30a, 31 a, 32a; 37, 38, 38, 40) miteinander verbunden sind.

**18.** Vorrichtung nach Anspruch 17, ferner umfassend einen Luftdetektor (34, 35), der zwischen dem Ventilmittel (28, 28a; 36, 41) und dem Ende der Blutentnahmeleitung (29) und/oder der Blutrückflussleitung (30) angeordnet ist, das mit dem Gefäßzugang eines Patienten zu verbinden ist.

## Revendications

**1.** Appareil pour un traitement sanguin extracorporel comprenant :

• un dialyseur (11) présentant un premier et un second compartiments (12, 13) séparés par une membrane semi-perméable ;
• un circuit sanguin présentant :

une ligne de retrait du sang (29) pour raccorder, dans une première condition de fonctionnement, une entrée du premier compartiment (12) à un accès vasculaire d'un patient à un premier point de raccordement (5), et
une ligne de retour du sang (30) pour raccorder, dans la première condition de fonctionnement, une sortie du premier compartiment (12) à l'accès vasculaire du patient à un second point de raccordement (6), dans lequel les premier et second points de raccordement sont espacés dans le sens du flux ;

• un circuit de liquide de dialyse présentant :

une ligne d'alimentation pour alimenter un liquide de dialyse dans une entrée du second compartiment (13), et
une ligne de résidus pour drainer un liquide résiduel d'une sortie du second compartiment (13),

l'appareil étant **caractérisé en ce qu'**il comprend :

• un moyen d'inversion (28) pour commuter le raccordement de la ligne de retrait du sang (29) et de la ligne de retour du sang (30) à l'accès vasculaire de sorte que, dans une seconde condition de fonctionnement, le premier point de raccordement (5) est en communication fluidique avec la sortie du premier compartiment (12) et le second point de raccordement (6) est en communication fluidique avec l'entrée du premier compartiment (12) ;
• un moyen de mesure (20) pour mesurer, dans la première condition de fonctionnement, une première valeur (Cd norm) d'une propriété dans le liquide résiduel, et pour mesurer, dans la seconde condition de fonctionnement, une seconde valeur (Cd rev) de la propriété dans le liquide résiduel, la propriété étant essentiellement proportionnelle à la concentration d'une substance dans le liquide résiduel ; et
• un moyen de calcul pour calculer un débit de fluide (Qa) dans l'accès vasculaire à partir des première et seconde valeurs mesurées (Cd norm, Cd rev) de la propriété dans le liquide résiduel.

**2.** Appareil selon la revendication 1, dans lequel la substance est sélectionnée dans le groupe comprenant l'urée, la créatinine, la vitamine B12, la bêta-2-microglobuline et le glucose.

**3.** Appareil selon la revendication 1, dans lequel la substance est un ion sélectionné dans le groupe comprenant $Na^+$, $Cl^-$, $Mg^{++}$, $Ca^{++}$, $HCO_3^-$, l'ion acétate, ou toute combinaison de ceux-ci.

4. Appareil selon l'une des revendications 1 à 3, dans lequel la propriété est la conductivité du liquide de dialyse.

5. Appareil selon l'une des revendications 1 à 3, dans lequel la propriété est la différence de la concentration de la substance entre la sortie et l'entrée du dialyseur (11).

6. Appareil selon l'une des revendications 1 à 3, dans lequel la propriété est soit la concentration de la substance dans le sang, soit l'efficacité corporelle totale relative (Kwb/V) définie en tant que rapport entre la clairance corporelle totale Kwb et le volume de distribution de l'urée V.

7. Appareil selon l'une des revendications 1 à 6, dans lequel le calcul du débit (Qa) du liquide dans l'accès vasculaire prend en compte un paramètre (K, Keff) indicateur du transfert de la substance à travers la membrane semi-perméable (14).

8. Appareil selon la revendication 7, dans lequel le paramètre indicateur du transfert de la substance à travers la membrane semi-perméable (14) est la clairance (K) du dialyseur (11) pour la substance.

9. Appareil selon la revendication 8, dans lequel le débit (Qa) du liquide dans le tube est calculé en utilisant l'équation :

$$Cd\ norm\ /\ Cd\ rev = 1 + K/Qa$$

10. Appareil selon la revendication 7, dans lequel le tube est un accès au flux sanguin pour l'hémodialyse tel qu'un shunt artérioveineux ou une fistule, et dans lequel le paramètre indicateur du transfert de la substance à travers la membrane semi-perméable (14) est la clairance de dialyseur effective Keff, qui prend en compte une recirculation cardiopulmonaire.

11. Appareil selon la revendication 10, dans lequel le débit (Qa) du liquide dans l'accès au flux sanguin est calculé en utilisant l'équation :

$$Cd\ norm\ /\ Cd\ rev = 1 + Keff/Qa$$

12. Appareil selon la revendication 11, dans lequel la clairance de dialyseur effective Keff est calculée en utilisant l'équation :

$$Keff = Qd * Cd / Cs$$

où Qd est le débit du liquide de dialyse en aval du dialyseur (11), Cd est la concentration de la substance dans le liquide de dialyse en aval du dialyseur (11), et Cs est la concentration de la substance dans le sang veineux systémique.

13. Appareil selon la revendication 12, comprenant en outre des moyens pour mesurer la concentration (Cs) de la substance dans le sang veineux systémique, comportant :

• des moyens (8, 8a) pour arrêter le flux sanguin dans le circuit sanguin pour une durée suffisante pour permettre à la circulation cardiopulmonaire de s'équilibrer et arrêter le flux de liquide de dialyse ;
• des moyens (8, 8a) pour démarrer le flux sanguin dans le circuit sanguin avec une vitesse lente pour remplir la ligne artérielle avec du sang frais avant la mesure ;
• des moyens (20) pour mesurer la concentration équilibrée de la substance dans le liquide résiduel à un débit lent ou en ultrafiltration isolée.

14. Appareil selon la revendication 12, comprenant en outre des moyens pour évaluer la concentration (Cs) de la substance dans le sang veineux systémique, comportant :

• des moyens pour calculer une masse l'urée du corps entier (Murée) dans le corps du patient,
• des moyens pour évaluer ou mesurer le volume de distribution (V) de l'urée dans le corps du patient ;

• des moyens pour évaluer la concentration (Cs) de la substance dans le sang en divisant la masse de l'urée du corps entier par le volume de distribution.

**15.** Appareil selon l'une des revendications 1 à 14, comprenant en outre :

• des moyens (8, 8a) pour modifier le débit sanguin (Qb) ;
• des moyens (20) pour surveiller la concentration de ladite substance dans le liquide résiduel ;
• des moyens pour détecter une possible recirculation dans la fistule dans la première position en mettant en corrélation un changement dans ladite concentration avec un changement du débit sanguin.

**16.** Appareil selon la revendication 15, comprenant des moyens (8, 8a) pour diminuer le débit sanguin et des moyens (20) pour surveiller une diminution correspondante de la concentration de la substance, l'absence d'une telle diminution étant indicatrice d'une recirculation dans la fistule.

**17.** Appareil selon l'une des revendications 1 à 16, dans lequel le moyen d'inversion comprend un moyen de vanne (28, 28a ; 36, 41) raccordé à la ligne de retrait du sang (29) et à la ligne de retour du sang (30) pour inverser de manière sélective le raccordement de la ligne de retrait du sang (29) et la ligne de retour du sang (30) à l'entrée et à la sortie du premier compartiment (12) du dialyseur (11), le moyen de vanne (28, 28a ; 36, 41) comprenant deux orifices d'entrée et deux orifices de sortie (29a, 30a, 31 a, 32a ; 37, 38, 38, 40) et un élément de vanne (33, 33a ; 43) qui est agencé de telle manière qu'il prend de manière sélective une position de repos dans laquelle tous les quatre orifices d'entrée et de sortie (29a, 30a, 31 a, 32a ; 37, 38, 38, 40) sont interconnectés.

**18.** Appareil selon la revendication 17, comprenant en outre un détecteur d'air (34, 35) agencé entre le moyen de vanne (28, 28a ; 36, 41) et l'extrémité à raccorder à l'accès vasculaire d'un patient d'au moins l'une de la ligne de retrait du sang (29) et de la ligne de retour du sang (30).

Fig.1

Fig.2

## Fig.3

$$Cb - Cb\frac{K}{Qb}$$

$Cs$ $V$

$Qco-Qa$ 21

23

$Qa-Qb$

27

22

26 24 $Qco$ $Ca$ 25

$Qa$

8 5 $Cb$ $Cd$ 19 20

$Qb$ $Qd$

11

## Fig.4

$$Cb - Cb\frac{K}{Qb}$$

$Cs$ $Q$ $Cb$

$Qco-Qa$ $Qa$ 6

27 $Cb$

$Qa+Qb$

$Ca$ $Qco$ $Ca$ 5 P

$Qa$

8 $Cb$ $Cd$ 19 20

$Qb$ $Qd$

11

## Fig.5

## Fig.6

Dialysate urea concentration (mmol/l)

Period with reversed lines

Treatment time (min)

Fig. 7

Fig. 8

Fig. 9

## Fig. 10

## Fig. 11

Relative whole body efficiency K/V (per min)

Period with reversed lines

Treatment time (min)

36

41

*Fig. 12*

44   45   43

42   46a

*Fig. 13*

37

47

36

39   48   46

40

43   41

38

*Fig. 14*

22

**EP 1 124 599 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5685989 A
- US 5595182 A
- US 5453576 A
- US 5510716 A
- US 5510717 A
- US 5312550 A
- EP 0773035 A
- US 5605630 A
- US 5894011 A
- WO 9855166 A
- EP 658352 A
- WO 9929355 A

**Non-patent literature cited in the description**

- **JOHN T. DAUGIRDAS ; TODD. S ING.** Handbook of Dialysis. 1994, 18